Europäisches Patentamt

European Patent Office

Office européen des brevets

(19)

(11) Publication number: **0 191 607**
A2

# EUROPEAN PATENT APPLICATION

(12)

(21) Application number: 86300828.0

(22) Date of filing: 06.02.86

(51) Int. Cl.⁴: **C 12 N 1/14,** C 12 N 1/38, A 01 G 1/04

(30) Priority: 09.02.85 GB 8503383

(43) Date of publication of application: 20.08.86 Bulletin 86/34

(84) Designated Contracting States: **AT BE DE FR GB IT LU NL SE**

(71) Applicant: **BP Chemicals Limited, Belgrave House 76 Buckingham Palace Road, London, SW1W 0SU (GB)**
Applicant: **THE VICTORIA UNIVERSITY OF MANCHESTER, Oxford Road, Manchester. M13 9PL (GB)**

(72) Inventor: **Moore, David Department of Botany, University of Manchester Oxford Road, Manchester, M13 9PL (GB)**
Inventor: **Trinci, Anthony Peter Joseph Department of Botany, University of Manchester Oxford Road, Manchester, M13 9PL (GB)**

(74) Representative: **Fawcett, Richard Fennelly et al, BP INTERNATIONAL LIMITED Patents Division Chertsey Road, Sunbury-on-Thames Middlesex, TW16 7LN (GB)**

(54) Improvements in the culture of fungi.

(57) A process for the improved culturing of filamentous basidiomycete fungi is provided. The improvement lies in adding to the culture medium a polymer additive which reduces the aggregation of the filaments of fungi. Preferred polymer additives are polyacrylate and polymethacrylate thickening agents.

EP 0 191 607 A2

1

## IMPROVEMENTS IN THE CULTURE OF FUNGI

The present invention relates to additives which may be used to improve the growth rate of fungal cells cultured in industrial fermentation apparatus and processes which use such additives. More particularly the invention relates to the use of additives which prevent aggregation and subsequent inhibition of the growth of filamentous fungi.

Fungi exist in a wide range of morphologies ranging from simple unicellular species such as yeast up to complex multicellular organisms for example mushrooms and the like. A common vegetative unit of many fungi is the hyphal filament, generally taking the form of a microscopic fibre, which may be a single cell or a cell interrupted at intervals by incomplete cross walls known as septa.

Fungi can be cultured in a variety of ways including submerged culture in a liquid medium containing essential nutrients. When fungi of the filamentous type grow in submerged liquid culture, under conditions typically found in industrial fermenters, the growth is in general three dimensional and initially follows exponential growth kinetics. However in many cases such growth quickly leads to the formation of structures resulting from the interweaving of individual growing hyphal filaments. As growth proceeds, interweaving increases rapidly and prevents separation of the individual hyphal filaments by diffusion processes. At the limit, the interwoven structure takes on the appearance of a solid pellet, crust or mat which can adhere to the sides of the reaction vessel or any ancillary agitation equipment present in the vessel.

1

Formation of fungal pellets by this mechanism can be a disadvantage when operating an industrial fermenter. Thus as the pellet is formed by interweaving of the hyphal filaments, egress of nutrients and oxygen to those filaments at the centre of the pellet is progressively restricted until, at the limit, they are starved. As the level of nutrient and oxygen reaching those filaments at the centre of the pellet decreases so the rate of growth of these filaments fall off and the rate of total growth of the fungal culture deviates from the theoretical maximum. In extreme cases, growth in the centre of the pellet stops completely and occurs only amongst those filaments on the surface of the pellet. When this occurs the productivity of the fermenter drops substantially.

A method of reducing the aggregation of the ascomycete fungus Aspergillus niger has been reported in Trans. Br. mycol soc. 81(2) 408 (1983). Experiments reported in this reference show that when Aspergillus niger was grown in submerged shaken flasks in the presence of a polyacrylate thickening agent such as Junlon PW 110 or PW 111 (Junlon is a trade name) disperse filamentous growth could be achieved over a range of conditions. Previously careful control was required to produce filamentous growth in preference to pellet formation. This work confirms earlier observations (Biotechnology and Bioengineering Vol XV, Pages 845-859 (1973)) that polymers of acrylic acid enhanced the growth rate of the same fungus.

Another class of fungi, the basidiomycetes, also show a tendency to undergo aggregation. Basidiomycetes represent about one third of the known species of fungi and are considered to be the most advanced of the higher fungi. Many basidiomycetes are known to produce secondary metabolites which have antibiotic activity. For example Lentinus edodes produces lenthionine,1,2,3,5,6-pentathiepane which shows strong antibiotic activity against a range of bacteria and fungi.

Polyacetylenic compounds which have antibiotic activity against bacteria occur in the following genera: Aleurodiscus, Clitocybe, Daedalea, Marasmius, Merulius, Pleurotus, Polyporus, Poria, Psathyrella and Tricholoma. Certain basidiomycetes produce

0191607

anti-fungal compounds, examples of these include, <u>Lentinus</u> <u>edodes</u> and <u>Coprinus</u> <u>comatus</u>.

Apart from their use in producing secondary metabolites there is great commercial potential for the production of basidiomycete biomass. Thus the total world production of edible mushroom biomass was in 1975 well over 1 million tons. About half the commercial mushrooms are sold processed rather than fresh. Mushrooms are a good source of several vitamins including thiamine, riboflavin, niacin, biotin and ascorbic acid. The most important cultivated mushroom in Japan is the basidiomycele <u>Lentinus</u> <u>edodes.</u> Basidiomycete fungi which are able to complex and remove metal ions e.g. lead, gold, silver, tin and uranium ions, from waste water streams can also be grown by this method.

Finally, there are many basidiomycetes with a wood habitat, which are lignin degrading, for example <u>Pleurotus</u> <u>ostreatus</u> and <u>Phanerochaete</u> <u>chrysosporium</u> and these have great potential for the treatment of industrial wastes.

Even though many basidiomycete fungi have such industrial potential, little work has been done on their cultivation by fermentation since they have a greater tendency than any other fungi to aggregate in agitated liquid culture.

A method of promoting the growth of such fungi is disclosed in GB 2032456. This patent teaches that the growth of such fungi can be promoted by adding to the culture medium between 0.01 and 10ppm of a straight chain aliphatic alcohol of general formula $C_nH_{2n+1}OH$ wherein n is an integer of 26 to 36.

It has now been found that in addition to these alcohols it is possible to use certain anionic polymer additives to promote the growth rate of basidiomycete fungi by reducing the tendency of the hyphal filaments of the fungi to aggregate. Thus the anionic polymer additive can reduce the size of pellets produced under given set of culture conditions or in some circumstances prevent aggregation completely thereby producing cultures of the basiomycete fungus comprising unaggregated hyphal filaments. The hyphal filaments are either single cells or single cells interrupted by septa.

Accordingly the present invention comprises a process for the cultivation of a basidiomycete fungus of the filamentous type which process comprises cultivating the basidiomycete fungus in a culture medium under conditions which cause the basidiomycete fungus to grow characterised in that the culture medium contains an anionic polymer additive to reduce aggregation of the filaments of the basidiomycete fungus produced during the cultivation.

The use of an anionic polymer additive has the additional advantage that it prevents adherence of the fungi to the internal surface of the fermenter and hence allows facile removal of the fungi when necessary. Such an effect can obviously be used to especial advantage in continuously operated fermenters where it is necessary to continually remove the fungus as the culture medium becomes exhausted. Use of a polymer additive therefore allows basidiomycete fungi to be grown simply and efficiently in industrial fermenters.

The basidiomycete fungi which show improved growth characteristics when cultured in a medium containing a polymer additive are the basidiomycetes of the filamentous type. These include those basidiomycetes commercially cultivated on a solid medium for foodstuffs such as members of the <u>Agaricus</u>, <u>Lentinus</u>, <u>Volvariella</u> and <u>Pleurotus</u> genera and edible, non-commercially cultivated species of the <u>Coprinus</u> genus. In addition the method may be applied to non edible species such as for example the <u>Sporotrichum</u> or <u>Phanerochaete</u> genera which are able to degrade waste material or able to produce enzymes, chemicals or secondary metabolites which have commercial value.

The basidiomycete fungi may also be imperfect fungi for which no sexual stage has, as yet, been demonstrated.

The basidiomycete fungi described herein may be grown in a variety of culture media, the exact nature of which will be familiar to those skilled in the art. Thus a simple nutrient solution can be used; for example one containing a carbon source (e.g. glucose and the like), a sulphate source, phosphate to stabilise the pH, sources of organic nitrogen, magnesium and any trace elements required by the specific fungi. On the other hand the medium may contain extracts derived from microbial or plant sources which contain mixtures of potential nutrients. Examples are meat extracts, yeast extracts, malt extracts, worts, potato extracts, oak bark extracts, soybean flower, rice bran, wheat bran, mycological peptones and hydrolysed casein.

The culture of the basidiomycete fungus is preferably carried out at temperatures in the range of 15 to 60°C, preferably in the range 20 to 40°C. The culture is suitably carried out under substantially aerobic conditions.

As stated before an advantage of this invention is that basidiomycete fungi can be grown in conventional fermenters without the need for using any specifically designed modifications. However the fermenter should consist of a vessel, a stirrer device an aeration device and may also be equipped with apparatus for controlling pH and oxygen and carbon dioxide levels, for dosing the vessel with metabolites and means for withdrawing the fermenter contents.

The polymer additives which are used to reduce aggregation of the hyphal filaments are ones which are anionic (i.e. carry a negative charge) under the culture conditions. The polymer additive can for example comprise an inorganic or organic backbone onto which are joined negatively charged hydrophilic groups. The negatively charged hydrophilic groups can be for example carboxylate ($-CO_2^-$) groups, phosphate groups, sulphate groups and the like. Preferably, the hydrophilic groups are carboxylate groups as in certain commercially available polyacrylates, or polymethacrylates. For example polymer additives having physical properties similar to those polyacrylate thickeners sold under the trade names Junlon, Carbapol or Hostacerin are particularly effective. The polymer additive can be one which is degraded by the fungi during the process but is preferably not as in this case it may not always be possible to prevent the aggregation.

The anionic polymer additive is used in amounts so as to conveniently constitute less than 20% by weight of the culture medium preferably in amounts in the range 0.001 to 5% by weight.

The invention will now be illustrated by reference to the following examples.

Example 1

A fermenter was charged with a culture medium consisting of 1% hydrolysed casein, 10 millimoles of glucose, 30 millimoles of ammonium chloride, 0.145% di-sodium hydrogen phosphate, 0.135% potassium dihydrogen phosphate 0.029% sodium sulphate and 1 mg of thiamine hydrochloride in 1 litre of distilled water. The basidiomycete Coprinus cinereus was added to the culture medium together with 0.05% by wt Junlon PW110 and growth allowed to occur at 37°C and with an air flow through the fermenter of 5 litre/minute until the nutrients were exhausted. A homogenous dispersion of the fungus in the form of unaggrgated hyphal filaments was obtained at the end of the experiment with an average yield of 70-80 mg dry weight of basidiomycete per 100 mls of culture medium.

Comparative Test A

Example 1 was repeated in the absence of Junlon PW110 the product consisted of aggregates which strongly adhered to the fermenter walls and stirrer and proved difficult to remove. The average yield of basidiomycete was 60 mg dry weight per 100 mls of culture medium.

Example 2

Example 1 was repeated using 0.1% by weight Junlon PW110 instead of 0.05%. The product was again a dispersion of unaggregated hyphal filaments which was easy to remove from the reactor and the basidiomycete yield in the range 70-80 mg dry weight per 100 ml of culture medium.

Examples 3-11 and Comparative Tests B-L

A 250ml shake flask was charged with a medium comprising 20mls of yeast mycological peptone (prepared by mixing and sterilising 10g glucose, 5g of mycological peptone, 1.5g of yeast extract and 1 litre of distilled water) together with 0.2% (w/v) of the

0191607

appropriate additive and 1ml of a spore suspension of Phanerochaete chrysosporium ($5x10^5$ spores per ml).

The shake flasks were shaken on a rotary shaker (New Brunswick Inc) at 200 rpm and at 37°C for 30 hours. The morphology of the culture was inspected either visually or microscopically as appropriate.

Example 9 and Comparative Tests B and C illustrate the problems which can occur when biodegradable materials such as cellulose are used. In this case whether or not the anionic polymer thickener works depends on the culture conditions, molecular weight of the thickener etc.

**EFFECT OF ADDITIVE ON GROWTH OF PHANEROCHAETE CHRYSOSPORIUM**

| Example No or Comparative Test | Additive | Type of Additive | Culture Morphology After Incubation at 37°C for 30hrs (PH = 6.5) |
|---|---|---|---|
| 3 | Junlon PW110 | Anionic – polyacrylate | Dispersed Filamentous Growth |
| 4 | Junlon PW150 | Anionic – polyacrylate | Dispersed Filamentous Growth |
| 5 | Hostacerin | Anionic – polyacrylate | Dispersed Filamentous Growth |
| 6 | Texipol HSM2 | Anionic – polyacrylate | Small Pellets |
| 7 | Manucol Ester E/RE | Anionic – algimate | Large and Small Pellets |
| 8 | Manucol Ester M | Anionic – algimate | Large and Small Pellets |
| 9 | Cellobond 45000 | Anionic – cellulose | Large and Small Pellets |
| B | Cellobond 7000 | Anionic – cellulose | Large Single Pellet |
| C | Cellobond 300 | Anionic – cellulose | Large Single Pellet |
| 10 | Jaypol A140 | Anionic – polyacrylate | Small Pellets |
| 11 | Jaypol 40 | Anionic – polyacrylate | Small Pellets |
| D | Radioquat 6470 | Cationic – quat ammonium | Clumped Ungerminated Spores |
| E1 | Texipol HSED | Cationic – polyacrylate | Clumped Ungerminated Spores |
| F | Montanox 20 | Nomionic – polysorbate | Crust and Smal Pellets |
| G | Avicel CL611 | Nonionic – cellulose | Crust and Small Pellets |
| H | Nymcel | Nonionic – cellulose | Crust and Large Pellets |
| I | Gelcarin CIC | Nonionic – carrogeenan | Crust and Large Pellets |
| J | Polyvinyl Alcohol 205 | Nonionic | Large Single Pellet |
| K | Polyvinyl Alcohol 105 | Nonionic | Large Single Pellet |
| L | No Additive | – | Crust and Large Pellet |

0191607

Claims:

1. A process for the cultivation of a basidiomycete fungus of the filamentous type, which process comprises cultivating the filamentous basidiomycete fungus in a culture medium, under conditions which cause the basidiomycete fungus to grow, characterised in that the culture medium contains an anionic polymer additive to reduce aggregation of the filaments of the basidiomycete fungus produced during the cultivation.

2. A process as claimed in Claim 1 characterised in that the anionic polymer additive comprises an organic or inorganic backbone onto which are joined negatively charged hydrophilic groups.

3. A process as claimed in Claim 2 characterised in that the hydrophilic groups are selected from carboxylate groups, phosphate groups and sulphate groups.

4. A process as claimed in Claim 1 characterised in that the anionic polymer additive is a polyacrylate or polymethacrylate.

5. A process as claimed in claim 4 characterised in that the anionic polymer additive is a polyacrylate having physical properties which are the same or similar to those polyacrylate thickeners sold under the trade names Junlon, Carbapol or Hostacerin.

6. A process as claimed in any of the proceeding claims characterised in that the basidiomycete fungus is selected from members of the Agaricus, Lentinus, Volvariella, Pleurotus, Coprinus, Sporotrichum and Phanerochaete genera of fungi.

7. An anionic polymer additive for use in a process for the cultivation of a basidiomycete fungus as defined in claim 1.

8.    A dispersion comprising a culture of unaggregated hyphal filaments of a basidiomycete fungus in a medium, which has been used to cultivate the basidiomycete fungus, and an anionic polymer additive.

9.    A culture of a basidiomycete fungus comprising unaggregated hyphal filaments which hyphal filaments comprise single cells or single cells interrupted by septa.

10.    A culture of a basidiomycete fungus as claimed in claim 8 wherein the basidiomycete fungus is a member of a genera selected from <u>Agaricus</u>, <u>Lentinus</u>, <u>Volvariella</u>, <u>Pleurotus</u>, <u>Coprinus</u>, <u>Sporotrichum</u> and <u>Phanerochaete</u>.

11.    A medium for use in cultivating a basidiomycete fungus comprising a culture medium and an anionic polymer additive, the anionic polymer comprising less than 20% by weight of the culture medium.